# EUROPEAN PATENT APPLICATION

(11) **EP 2 754 402 A2**
(43) Date of publication of application: **16.07.2014**
(21) Application number: 12829870.0
(22) Date of filing: 05.09.2012
(51) Int. Cl.: A61B 17/34, B25J 18/06

(54) **MINIMALLY INVASIVE SURGICAL INSTRUMENT HAVING JOINT SECTION COMPRISING SPHERICAL COMPONENTS**

(30) Priority: 05.09.2011 KR 20110089854
(71) Applicant: Movasu, Inc., Seoul 110-450 (KR)
(72) Inventor: JEONG, Chang Wook, Seoul 110-450 (KR); SIN, Chun Chol, Seoul 110-450 (KR); KIM, Sung Ryul, Seoul 110-450 (KR); KIM, Hyung Tae, Seoul 110-450 (KR)
(74) Representative: Zardi, Marco
(86) International application number: PCT/KR2012/007093
(87) International publication number: WO 2013/036023

(57) **Abstract**

The present invention relates to a minimally invasive surgical instrument having a joint section comprising spherical components. According to one embodiment of the present invention, a minimally invasive surgical instrument comprises a shaft, a joint section connected to one end of the shaft, and an end effector which is connected to the joint section and thus is capable of articulatory movement, wherein the joint section comprises multiple spherical components and multiple joint links, and at least some of the multiple joint links move in the pitch direction or in the yaw direction in accordance with the shape of slots formed in the spherical components housed therein, enabling the end effector to move in the pitch or the yaw direction.

## Description

### FIELD OF THE INVENTION

The present invention relates to a minimally invasive surgical instrument having a joint unit comprising spherical components.

### BACKGROUND

Minimally invasive surgery is a surgical approach that involves the use of instruments inserted through several tiny incision openings to perform a surgery causing minimal tissue trauma in human or animal bodies.

The minimally invasive surgery relatively reduces changes in metabolism of a patient in the period of post-surgical care, so it facilitates rapid recovery of the patient. Therefore, the minimally invasive surgery shortens the length of hospitalization of the patient after the surgery and allows the patient to return to normal physical activities in a short period of time. In addition, the minimally invasive surgery causes less pain and leaves fewer scars on the patient's body after the surgery.

One of the general forms of the minimally invasive surgery is endoscopy. Among the others, a laparoscopy that involves minimally invasive inspection and operation inside abdominal cavity is known as the most general form of endoscopy. To operate a standard laparoscopic surgery, the abdomen of the patient is insufflated with gas and at least one small incision is formed to provide an entrance for laparoscopic surgical instruments, through which a trocar is inserted. When performing the surgery, it is general that a user puts the laparoscopic surgical instruments into a surgical site or the like through the trocar, and manipulates (or controls) the instruments from the outside of abdominal cavity. In general, the laparoscopic surgical instruments include a laparoscope (for observation of a surgical site) and other working tools. Herein, the working tools are similar to the conventional tools used for small incision surgery, except that the end effector or working end of each tool is separated from its handle or the like by a shaft. For instance, the working tools may include a clamp, a grasper, scissors, a stapler, a needle holder, and so forth. Meanwhile, the user monitors the procedure of the surgery through a monitor that displays the images of the surgical site which are taken by the laparoscope. The endoscopic approaches similar to the above are broadly used in retroperitoneoscopy, pelviscopy, arthroscopy, cisternoscopy, sinuscopy, hysteroscopy, nephroscopy, cystoscopy, urethroscopy, pyeloscopy, and so on.

The inventor(s) has developed various minimally invasive surgical instruments useful for the above-mentioned minimally invasive surgeries and has already disclosed the features of the structures and effects of the same in Korean Patent Application Nos. 2008-51248, 2008-61894, 2008-79126 and 2008-90560, the contents of which are incorporated herein by reference in its entirety. Additionally, the inventor(s) have also introduced a minimally invasive surgical instrument with improved functionality, which is more advantageous for users and patients, in Korean Patent Application Nos. 2010-115152, 2011-3192, 2011-26243 and 2011-29771, the contents of which are incorporated herein by reference in its entirety.

Herein, the inventor(s) now present a minimally invasive surgical instrument having a more improved joint unit than those disclosed in the above Korean applications or others.

### SUMMARY OF THE INVENTION

One object of the present invention is to provide a minimally invasive surgical instrument having a short-length joint unit.

Another object of the invention is to provide a minimally invasive surgical instrument in which joint motion is smoothly carried out in a joint unit.

Yet another object of the invention is to provide a minimally invasive surgical instrument in which the joint motion state of an end effector may be firmly fixed.

Still another object of the invention is to provide a minimally invasive surgical instrument to allow an end effector to smoothly carry out rolling motion.

Still yet another object of the invention is to provide a minimally invasive surgical instrument to allow an end effector to smoothly carry out rolling motion when it has carried out joint motion or even when the state of the joint motion has been fixed.

According to one aspect of the invention to achieve the objects as described above, there is provided a minimally invasive surgical instrument comprising a shaft; a joint unit being connected to one end of the shaft; and an end effector being connected to the joint unit and capable of carrying out joint motion thereby, wherein the joint unit comprises a plurality of spherical components and a plurality of joint links, and wherein at least some of the plurality of joint links operate in a pitch direction or yaw direction according to the shape of slots formed in the spherical components housed therein to allow the end effector to operate in the pitch direction or yaw direction.

In addition, there may be provided other configurations to implement this invention.

According to the invention, there is provided a minimally invasive surgical instrument having a short-length joint unit.

According to the invention, there is provided a minimally invasive surgical instrument in which joint motion is smoothly carried out in a joint unit.

According to the invention, there is provided a minimally invasive surgical instrument in which the joint motion state of an end effector may be firmly fixed.

According to the invention, there is provided a minimally invasive surgical instrument to allow an end effector to smoothly carry out rolling motion.

According to the invention, there is provided a minimally invasive surgical instrument to allow an end effector to smoothly carry out rolling motion when it has carried out joint motion or even when the state of the joint motion has been fixed.

### BRIEF DESCRIPTION OF THE DRAWINGS

Fig. 1 shows the overall appearance of a minimally invasive surgical instrument according to one embodiment of the invention.
Fig. 2 is a partially enlarged view of a joint unit 300 of the minimally invasive surgical instrument shown in Fig. 1.
Figs. 3 to 5 are partially enlarged views of the joint unit 300 of the minimally invasive surgical instrument shown in Fig. 1 being in a different state (i.e., having carried out joint motion in various directions).
Fig. 6 is a perspective view of some components such as an end effector 200 and a joint unit 300 of a minimally invasive surgical instrument according to one embodiment of the invention.
Fig. 7 is an exploded perspective view of the components of the minimally invasive surgical instrument shown in Fig. 6.
Fig. 8 is a cross-sectional view to show in more detail how the various components of the minimally invasive surgical instrument shown in Fig. 7 are connected.

### DETAILED DESCRIPTION OF THE PREFERRED EMBODIMENTS

In the following detailed description of the invention, references are made to the accompanying drawings that show, by way of illustration, specific embodiments in which the invention may be practiced. These embodiments are described in sufficient detail to enable those skilled in the art to practice the invention. It is to be understood that the various embodiments of the invention, although different from each other, are not necessarily mutually exclusive. For example, specific shapes, structures, or characteristics described herein may be implemented as modified from one embodiment to another embodiment without departing from the spirit and the scope of the invention. Furthermore, it shall be understood that the locations or arrangements of individual elements within each embodiment may be also modified without departing from the spirit and the scope of the invention. Therefore, the following detailed description is not to be taken in a limiting sense, and the scope of the invention is to be taken as encompassing the scope of the appended claims and all equivalents thereof. In the drawings, like reference numerals refer to the same or similar elements throughout the several views.

Hereinafter, preferred embodiments of the present invention will be described in detail with reference to the accompanying drawings to enable those skilled in the art to easily implement the invention.

Meanwhile, it should be understood that the term "connection" herein encompasses a direct connection or an indirect connection (i.e., via separate components) between mechanical or other types of components.

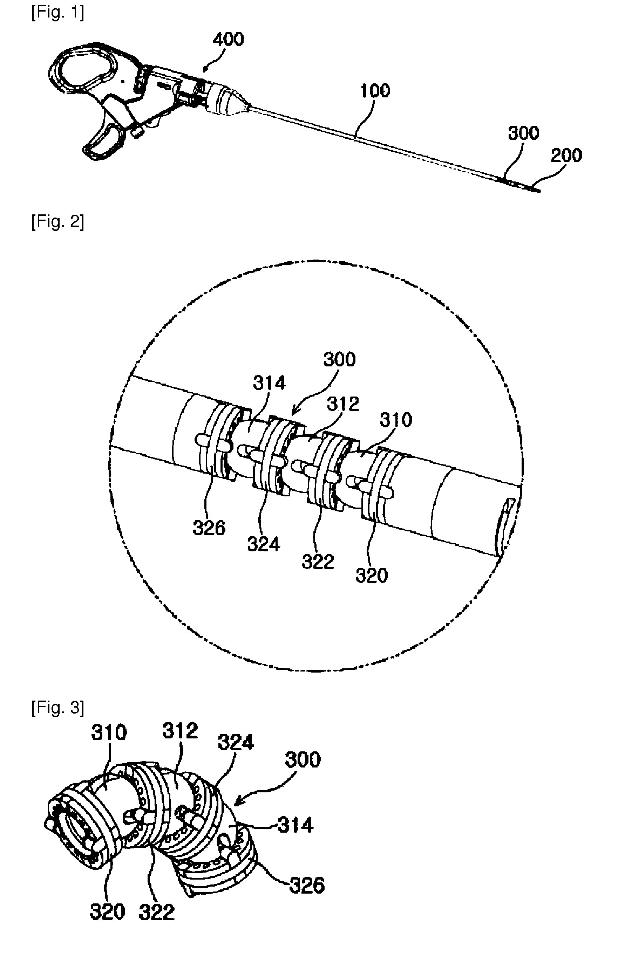

Fig. 1 shows the overall appearance of a minimally invasive surgical instrument according to one embodiment of the invention.

Reference will be made to Fig. 1. The minimally invasive surgical instrument may comprise a shaft 100; an end effector 200 being connected to one end of the shaft 100 to perform surgery by using surgical tools (not shown) or functioning itself as a surgical tool; a joint unit 300 to connect the shaft 100 and the end effector 200 and to provide the end effector 200 with joint functionality; and a handling unit 400 being connected to the other end of the shaft 100 and capable of being held and manipulated by a user.

First, the shaft 100 may include a cavity therein to support and pass at least one wire or torque transmission member, in the same manner as those of the minimally invasive surgical instruments disclosed in the aforementioned Korean patent applications. (The torque transmission member is mainly intended for the rolling motion of the end effector 200, while the shaft 100 may function itself as the torque transmission member in some cases.) The shaft 100 may comprise at least one segment as necessary. Further, the shaft 100 may comprise a bend in at least a part thereof.

Next, the end effector 200 may carry out joint motion, rolling motion, opening/closing motion and the like by the action of the at least one wire or torque transmission member passing from the handling unit 400 to the joint unit 300 via the shaft 100, in the same manner as those of the minimally invasive surgical instruments disclosed in the aforementioned Korean patent applications. The tip of the end effector 200 may be implemented in the form of a clamp, a grasper, a pair of scissors, a stapler, a needle holder, a hook-type electrode or the like.

Next, the joint unit 300 may act together with the at least one wire or torque transmission member to allow the end effector 200 to carry out joint motion, rolling motion and the like, in the same manner as those of the minimally invasive surgical instruments disclosed in the aforementioned Korean patent applications.

Finally, the handling unit 400 may control the joint motion, rolling motion, opening/closing motion and the like of the end effector 200 according to the user's manipulation, in the same manner as those of the minimally invasive surgical instruments disclosed in the aforementioned Korean patent applications. To allow for such control, the at least one wire or torque transmission member may be connected to the handling unit 400.

Fig. 2 is a partially enlarged view of the joint unit 300 of the minimally invasive surgical instrument shown in Fig. 1.

Reference will be made to Fig. 2. The joint unit 300 may comprise, for example, three spherical components 310, 312, 314 and four joint links 320, 322, 324, 326. As shown, two of the joint links 320, 322, 324, 326, which are disposed in sequence, may be connected to each other with the corresponding one of the spherical components 310, 312, 314 being housed therebetween. Each joint formed by two of the joint links 320, 322, 324, 326 and one of the spherical components 310, 312, 314 may serve as joints for pitch direction and yaw direction operations (PY or YP) of the end effector 200 according to the principle to be described below. Accordingly, the joint unit 300 may have a joint structure of PY-PY-PY or YP-YP-YP. (In connection with the features (particularly advantages) of the joint structure similar to the above, reference may be made to Korean Patent Application No. 2011-3192.)

Further, each of the joint links 320, 322, 324, 326 may provide a penetration passage (e.g., a penetration hole) or a similar binding site for a pitch wire (not shown) and/or a yaw wire (not shown) in the same manner as the joint links or connecting units disclosed in Korean Patent Application No. 2011-3192. Accordingly, the end effector 200 may carry out joint motion by the action of the wires in the joint unit 300.

Figs. 3 to 5 are partially enlarged views of the joint unit 300 of the minimally invasive surgical instrument shown in Fig. 1 being in a different state (i.e., having carried out joint motion in various directions). Referring to these views, it will be further discussed how the spherical components 310, 312, 314 and the joint links 320, 322, 324, 326 are connected.

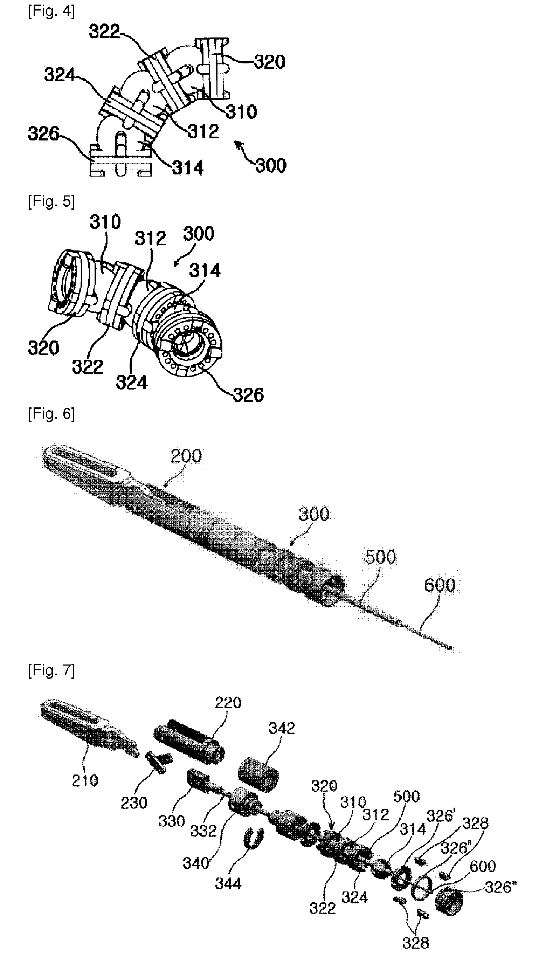

Fig. 6 is a perspective view of some components such as the end effector 200 and the joint unit 300 of the minimally invasive surgical instrument according to one embodiment of the invention. Further, Fig. 7 is an exploded perspective view of the components of the minimally invasive surgical instrument shown in Fig. 6.

Reference will be made to Fig. 6. As described above, the end effector 200 and the joint unit 300 may be connected to each other, and a torque transmission member 500 or an opening/closing wire 600 may be supported and passed through some part of the joint unit 300 (preferably the longitudinal central axis thereof) toward the end effector 200. Further, the opening/closing wire 600 may be supported and passed through a cavity or passage within the torque transmission member 500. In general, the basic configuration or function of the torque transmission member 500 or the opening/closing wire 600 is as described above. Meanwhile, particular reference may be made to Korean Patent Application No. 2011-29771 in connection with various kinds of the torque transmission member 500 which may be supported and passed within the bended or bendable components such as the shaft 100 and the joint unit 300.

Reference will be made to Fig. 7.

First, the end effector 200 may comprise a working end 210, a working end connecting unit 220 and an X-shaped link 230. The configuration and operating principle of the end effector 200 may be similar to those of the end effectors disclosed in the aforementioned Korean patent applications.

Further, the joint unit 300 may comprise the spherical components 310, 312, 314 and the joint links 320, 322, 324, 326 as described above.

Each of the spherical components 310, 312, 314 may provide a passage for the torque transmission member 500 or the opening/closing wire 600, preferably in the central axis thereof. Further, each of the spherical components 310, 312, 314 may comprise at least one, preferably a pair of slots on one hemisphere thereof facing the handling unit 400 to only allow one of the pitch direction and yaw direction operations of the corresponding joint link, and at least one, preferably a pair of slots on the other hemisphere thereof facing the end effector 200 to only allow the other operation of the corresponding joint link.

Next, the configuration of the joint links 320, 322, 324, 326 will be discussed with the example of the joint link 326. The joint link 326 may be configured to comprise two housing members 326', 326", a ring 326" and four bolster pieces 328. The housing members 326', 326" may be in the form of a ring or plate to provide a space for housing the spherical component 314, and may have on the periphery thereof two slots at the positions corresponding to the adjacent slots of the spherical component 314 and other two slots at the positions in the direction perpendicular thereto. By fitting each bolster piece 328 into each of the slots of the housing members 326', 326" and putting the ring 326" therearound (preferably capable of properly tightening the bolster pieces 328), the housing members 326', 326" may be coupled to each other and each of the bolster pieces 328 may be fixed to the housing members 326', 326".

Two of the four bolster pieces 328 (i.e., two disposed in the pitch direction) may be somewhat loosely fitted into the slots of the spherical component 314. (This configuration is intended to substantially guide the pitch direction operation of the spherical component 314 as will be described below.) The other two may fix the spherical component 314 by directly bolstering it. (This configuration is intended to prevent the yaw direction operation of the spherical component 314.) Thus, the corresponding bolster pieces 328 may allow the adjacent joint link 324 to operate together with the spherical component 314 only in the pitch direction when the joint link 324 is pulled by the pitch wire and to operate independently of the spherical component 314 only in the yaw direction when the joint link 324 is pulled by the yaw wire.

Each of the remaining joint links 320, 322, 324 may be configured in the same manner as described above with the joint link 326. It should be noted that each of the four bolster pieces of each joint link may preferably have a length and shape enough to bolster and fix the regions with no slots of the adjacent spherical component to prevent the spherical component from moving in the corresponding direction. Further, each of the remaining spherical components 310, 312 may be configured in substantially or exactly the same manner as the spherical component 314 except that the slots in the yaw direction face the handling unit 400 and the slots in the pitch direction face the end effector 200. Therefore, those skilled in the art may implement the joint unit 300 to have a joint structure of PY-PY-PY or YP-YP-YP as described above by only determining how the spherical components 310, 312, 314 are disposed in the joint unit 300. Meanwhile, it is apparent that the joint structure of the joint unit 300 may be further extended as the number of the spherical components or the like thereof is increased. It is also apparent that the joint structure of the joint unit 300 may be reduced as necessary.

Further, the joint unit 300 may further comprise an opening/closing wire fixing unit 330, an opening/closing wire cap 332, a rolling connecting unit 340, a holder 342 and a fastener 344. (Herein, all of those are deemed to be the components of the joint unit 300 for convenience, while some of those may be considered to belong to the end effector 200 in some point of view.)

One end of the opening/closing wire 600 may be fixed to the opening/closing wire fixing unit 330 via the opening/closing wire cap 332 as the opening/closing wire 600 has been extended out of the torque transmission member 500. Thus, when the opening/closing wire 600 is pulled, the opening/closing wire fixing unit 330 may act on the X-shaped link 230 connected thereto so that the working end 210 is closed. (Naturally, the configuration of the end effector 200 may be slightly changed so that the end effector 200 is opened when the opening/closing wire 600 is pulled.)

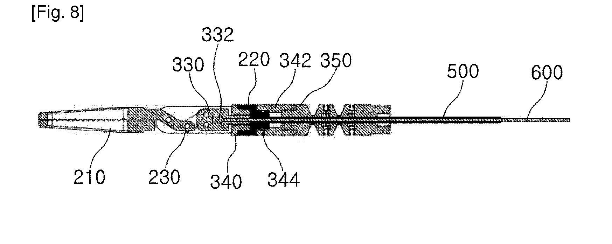

Further, the rolling connecting unit 340 may be connected to the torque transmission member 500 to rotate as the torque transmission member 500 carries out rolling motion. This rotation may cause the rotation of the working end connecting unit 220, which may be coupled to the rolling connecting unit 340 by means of the holder 342 as shown in Fig. 8, and further cause the rotation of the working end 210 connected thereto (i.e., the rolling motion of the end effector 200). (Fig. 8 is a cross-sectional view to show in more detail how the various components of the minimally invasive surgical instrument shown in Fig. 7 are connected.) In order to easily tighten or loosen the above coupling rendered by the holder 342, the fastener 344 which may be fitted in the connecting part between the working end connecting unit 220 and the holder 342 may be further employed as necessary.

### Applications

According to an application of the present invention, those skilled in the art may partially change the form and such of the handling unit or the like so that the wire or torque transmission member of the minimally invasive surgical instrument may be operated by an electric motor or the like of another motor-based system (not shown) such as a surgical robot, as necessary.

Although the present invention has been described in terms of specific items such as detailed elements as well as the limited embodiments and the drawings, they are only provided to help general understanding of the invention, and the present invention is not limited to the above embodiments. It will be appreciated by a person of ordinary skill in the art that various modifications and changes may be made from the above description.

Therefore, the spirit of the present invention shall not be limited to the above-described embodiments, and the entire scope of the appended claims and their equivalents will fall within the scope and spirit of the invention.

## Claims

1. A minimally invasive surgical instrument comprising:
a shaft;
a joint unit being connected to one end of the shaft; and
an end effector being connected to the joint unit and capable of carrying out joint motion thereby,
wherein the joint unit comprises:
a plurality of spherical components; and
a plurality of joint links, and
wherein at least some of the plurality of joint links operate in a pitch direction or yaw direction according to the shape of slots formed in the spherical components housed therein to allow the end effector to operate in the pitch direction or yaw direction.

2. A minimally invasive surgical instrument as claimed in Claim 1, wherein at least some of the plurality of spherical components comprise slots being formed in the pitch direction on one hemisphere thereof.

3. A minimally invasive surgical instrument as claimed in Claim 1, wherein at least some of the plurality of spherical components comprise slots being formed in the yaw direction on one hemisphere thereof.

4. A minimally invasive surgical instrument as claimed in Claim 1, wherein at least some of the plurality of spherical components comprise slots being formed in the pitch direction on one hemisphere thereof, and further comprise slots being formed in the yaw direction on the opposite hemisphere thereof.

5. A minimally invasive surgical instrument as claimed in Claim 1, wherein each of the plurality of spherical components comprises a passage in the central axis thereof for a torque transmission member for rolling motion of the end effector or an opening/closing wire for opening/closing motion of the end effector.

6. A minimally invasive surgical instrument as claimed in Claim 1, wherein at least some of the plurality of spherical components comprise bolster pieces.

7. A minimally invasive surgical instrument as claimed in Claim 6, wherein the bolster pieces guide or prevent the movement of the spherical components adjacent thereto.

8. A minimally invasive surgical instrument as claimed in Claim 6, wherein the bolster pieces are disposed on the periphery of the joint links comprising them.

9. A minimally invasive surgical instrument as claimed in Claim 8, wherein the bolster pieces are tightened by a ring to fix to the joint links.

10. A minimally invasive surgical instrument as claimed in Claim 9, wherein the joint links further comprise a pair of housing members, and the pair of housing members are coupled to each other by means of the ring.
